(19) Europäisches Patentamt
European Patent Office
Office européen des brevets

(11) Veröffentlichungsnummer : **0 112 292**
**B1**

(12) # EUROPÄISCHE PATENTSCHRIFT

(45) Veröffentlichungstag der Patentschrift : **29.07.87**

(21) Anmeldenummer : **83810577.3**

(22) Anmeldetag : **08.12.83**

(51) Int. Cl.[4] : **C 07 D249/08**, C 07 D233/60, C 07 D303/22, C 07 D303/34, A 01 N 43/50, A 01 N 43/64

(54) **Neue Wuchsregulatoren und Mikrobizide.**

(30) Priorität : **14.12.82 CH 7270/82**

(43) Veröffentlichungstag der Anmeldung : **27.06.84 Patentblatt 84/26**

(45) Bekanntmachung des Hinweises auf die Patenterteilung : **29.07.87 Patentblatt 87/31**

(84) Benannte Vertragsstaaten : **AT BE CH DE FR GB IT LI NL SE**

(56) Entgegenhaltungen :
**EP-A- 0 017 080**
**EP-A- 0 047 594**
**EP-A- 0 061 835**
**DD-A- 148 219**
**DE-A- 3 202 601**
**DE-A- 3 224 186**
**CHEMICAL ABSTRACTS, vol. 82, no. 17, 28. April 1975, Columbus, Ohio, USA, M.Z. KRIMER et al. "Synthesis of epoxides of citronellyl ethers of phenol and their juvenile hormonal activity", Seite 523, Abstract no. 112 168k**
**CHEMICAL ABSTRACTS, vol. 78, no. 11, 19. März 1973, Columbus, Ohio, USA, STOLNESCU "Dihydroxyamin ethers", Seite 413, abstract no. 71 427r**
***Die Akte enthält technische Angaben, die nach dem Eingang der Anmeldung eingereicht wurden und die nicht in dieser Patentschrift enthalten sind.***

(73) Patentinhaber : **CIBA-GEIGY AG**
**Klybeckstrasse 141**
**CH-4002 Basel (CH)**

(72) Erfinder : **Sturm, Elmar, Dr.**
**Klusstrasse 66**
**CH-4147 Aesch (CH)**
Erfinder : **Müller, Urs, Dr.**
**Schluchtstrasse 15**
**CH-4142 Münchenstein (CH)**
Erfinder : **Tobler, Hans, Dr.**
**Baselmattweg 157**
**CH-4123 Allschwil (CH)**

**EP 0 112 292 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 1-Fluor-1-aryloxy-2-azolylmethyl-2-alkanol-Derivate der nachstehenden Formel I, deren Säureadditionssalze, quaternäre Azoliumsalze und Metallkomplexe. Sie betrifft auch neue Oxirane der nachstehenden Formel II. Die Erfindung betrifft ferner die Herstellung dieser Substanzen sowie wuchsregulierende und mikrobizide Mittel, die als Wirkstoff mindestens eine dieser Substanzen enthalten.

Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen.

Bei den erfindungsgemässen Verbindungen handelt es sich um solche der allgemeinen Formel I

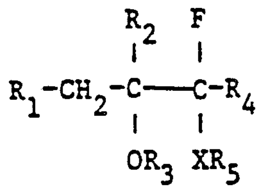

(I)

worin

$R_1$ für eine 1,2,4-Triazolyl- oder Imidazolylgruppe steht ;

$R_2$ $C_1$-$C_{12}$-Alkyl bedeutet ;

$R_3$ Wasserstoff, $C_1$-$C_{12}$-Alkyl, $C_2$-$C_4$-Alkenyl, $C_2$-$C_4$-Alkinyl oder gegebenenfalls durch $C_1$-$C_6$-Alkyl, $C_1$-$C_6$-Alkoxy, $C_1$-$C_6$-Haloalkyl, Halogen und/oder Cyano substituiertes Benzyl bedeutet ;

$R_4$ für Wasserstoff, Fluor oder $C_1$-$C_6$-Alkyl steht ;

$R_5$ für einen unsubstituierten oder substituierten Rest, ausgewählt aus der Reihe Phenyl, Naphthyl, Biphenyl, Benzylphenyl und Benzyloxyphenyl steht, wobei die Substituenten aus der Reihe Halogen, Cyano, $C_1$-$C_6$-Alkyl, $C_1$-$C_6$-Alkoxy, $C_1$-$C_6$-Haloalkyl, $C_1$-$C_6$-Alkylthio, $C_1$-$C_6$-Haloalkoxy, $C_1$-$C_3$-Haloalkylthio, Nitro und/oder Rhodano ausgewählt sind ; und

X Sauerstoff oder Schwefel bedeutet ; unter Einschluss der Säureadditionssalze, quaternären Azoliumsalze und Metallkomplexe.

Aus der Literatur sind 2-Hydroxypropyl-azol-Derivate als Fungizide beispielsweise aus der Europäischen Patentanmeldung EP-A-61 835 bekannt.

Unter dem Begrifff Alkyl selbst oder als Bestandteil eines anderen Substituenten sind je nach Zahl der angegebenen Kohlenstoffatome beispielsweise folgende Gruppen zu verstehen : Methyl, Ethyl, Propyl, Butyl, Pentyl, Hexyl, Heptyl, Octyl, Nonyl, Decyl, Undecyl, Dodecyl usw. sowie ihre Isomeren, wie z. B. Isopropyl, Isobutyl, tert.-Butyl, Isopentyl usw. Haloalkyl steht für einen einfach bis perhalogenierten Alkylsubstituenten, wie z. B. $CHCl_2$, $CHF_2$, $CH_2Cl$, $CCl_3$, $CH_2F$, $CH_2CH_2Cl$, $CHBr_2$ usw. vorzugsweise $CF_3$. Unter Halogen soll hier und im folgenden Fluor, Chlor, Brom oder Jod, vorzugsweise Fluor, Chlor oder Brom, verstanden werden. Naphthyl steht für α- oder β-Naphtyl, vorzugsweise α-Naphthyl. Der Ausdruck Haloalkoxy oder Haloalkylthio steht für einen Alkoxy- oder Alkylthiorest dessen Haloalkylanteil wie weiter oben unter Haloalkyl definiert ist, Alkenyl bedeutet z. B. Propenyl-(1), Allyl, Butenyl-(1), Butenyl-(2) oder Butenyl-(3), Alkinyl steht z. B. für Propionyl-(1) oder Propargyl. Rhodano steht für —SCN.

Die vorliegende Erfindung betrifft somit die freien organischen Verbindungen der Formel I in Form von Alkoholen oder Ethern sowie deren Säureadditionssalze, quaternäre Azoliumsalze und Metallkomplexe. Die freien Verbindungen sind bevorzugt, insbesondere die 1H-1,2,4-Triazole.

Beispiele salzbildender Säuren sind anorganische Säuren : Halogenwasserstoffsäurenwie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Oxalsäure, Ameisensäure, Benzolsulfonsäure, p-Toluolsulfonsäure oder Methansulfonsäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d. h. sie können ein oder mehrere organische Molekülanteile als Liganden erhalten. Komplexe mit den Metallen Kupfer, Zink, Mangan und Zinn sind bevorzugt.

Die Verbindungen der Formel I sind bei Raumtemperatur stabile Oele, Harze oder teilweise auch Feststoffe, die sich durch sehr wertvolle mikrobizide und wuchsregulierende Eigenschaften auszeichnen. Sie lassen sich auf dem Agrarsektor oder verwandten Gebieten präventiv und kurativ zur Bekämpfung

von pflanzenschädigenden Mikroorganismen und zur Regulierung des Pflanzenwuchses einsetzen, dabei sind die 1,2,4-Triazolyl(1)methylderivate im Umfang der Formel I bevorzugt. Die erfindungsgemässen Wirkstoffe der Formel I zeichnen sich durch eine sehr gute Verträglichkeit bei Kulturpflanzen aus.

Aufgrund ihrer ausgeprägten wuchsregulierenden und/oder mikrobiziden Wirkung sind folgende Untergruppen zunehmend bevorzugt :

Verbindungen der Formel I worin $R_2 C_1$-$C_6$-Alkyl bedeutet ; $R_3$ Wasserstoff, $C_1$-$C_6$-Alkyl, Allyl, Propargyl, Benzyl, 2,6-Dichlorbenzyl, 4-Chlorbenzyl, 4-Fluorbenzyl, 2,4-Dichlorbenzyl oder 2-Chlor-4-fluorbenzyl bedeutet ; $R_4$ für Wasserstoff oder Methyl steht ; $R_5$ Phenyl, 4-Halophenyl, 2,4-Dihalophenyl oder durch $CF_3$ substituiertes Phenyl steht und X Sauerstoff oder Schwefel bedeutet.

Verbindungen der Formel I worin $R_1$ für 1,2,4-Triazol steht ; $R_2$ für tert.-Butyl oder iso-Propyl steht ; $R_3$ Wasserstoff oder $C_1$-$C_5$-Alkyl bedeutet ; $R_4$ Wasserstoff oder Methyl bedeutet ; $R_5$ für 4-Halophenyl, 2,4-Dihalophenyl oder $4—CF_3—C_6H_5—$ steht und X Sauerstoff bedeutet.

Innerhalb der letzten Gruppe sind insbesondere die Alkohole ($R_3$ = H) bevorzugt.

Besonders bevorzugte Einzelsubstanzen sind z. B. :

1-Fluor-1-(4-chlorphenoxy)-2-(1H-1,2,4-triazol-1'-ylmethyl)-3,3-dimethyl-butan-2-ol,
1-Fluor-1-(4-fluorphenoxy)-2-(1H-1,2,4-triazol-1'-ylmethyl)-3,3-dimethyl-butan-2-ol,
1-Fluor-1-(2,4-dichlorphenoxy)-2-(1H-1,2,4-triazol-1'-ylmethyl)-3,3-dimethyl-butan-2-ol,
1-Fluor-1-(4-chlorphenoxy)-2-(1H-imidazol-1'-ylmethyl)-3,3-dimethyl-butan-2-ol.

Die Verbindungen der Formel I werden dadurch herstellt, dass man ein Oxiran der Formel II

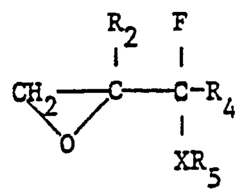

(II)

mit einem Azol der Formel III

$$M—R_1 \qquad \text{(III)}$$

zuerst zu einem Alkohol der Formel Ia,

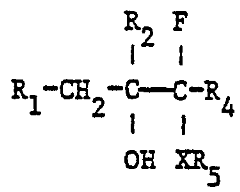

(Ia)

umsetzt und den Alkohol der Formel Ia, sofern ein Ether der Formel I hergestellt werden soll, auf übliche Art, z. B. durch Reaktion mit einer Verbindung der Formel IV

$$R_3—W \qquad \text{(IV)}$$

in einem Ether der Formel I überführt, wobei die Substituenten $R_1$ bis $R_5$ in den Formeln Ia, II, III und IV die unter Formel I angegebenen Bedeutungen haben, M für Wasserstoff oder bevorzugt ein Metallatom, insbesondere ein Alkalimetallatom wie Li, Na oder K steht, Hal Halogen, insbesondere Chlor oder Brom bedeutet, und W für OH oder eine übliche Abgangsgruppe steht, dabei sollen unter einer üblichen Abgangsgruppe hier und im folgenden Substituent wie z. B. Halogene : [wie Fluor, Chlor, Brom oder Jod, vorzugsweise Chlor oder Brom] ; Sulfonyloxygruppen, bevorzugt $—OSO_2—R_a$ ; Acyloxygruppen, bevorzugt $—OCO—R_a$ und Isoharnstoffreste, bevorzugt

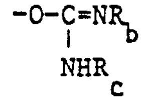

verstanden werden, wobei $R_a$, $R_b$ und $R_c$ unabhängig voneinander für $C_1$-$C_3$-Alkyl, $C_1$-$C_3$-Haloalkyl oder gegebenenfalls durch Halogen, Methyl, Nitro, Trifluormethyl und/oder Methoxy substituiertes Phenyl stehen.

Die Reaktion II mit III zu Ia wird gegebenenfalls in Gegenwart von Kondensationsmitteln oder von säurebindenden Mitteln durchgeführt. Als solche kommen organische und anorganische Basen in Betracht, z. B. tertiäre Amine wie Trialkylamine (Trimethylamin, Triethylamin, Tripropylamin usw.), Pyridin

und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$—nONa usw. In einigen Fällen kann es von Vorteil sein, wenn man das freie Azol III (M = Wasserstoff) zuerst, z. B. in situ mit einem Alkoholat, in das entsprechende Salz überführt und anschliessend in Gegenwart einer der genannten Basen mit dem Oxiran der Formel II umsetzt. Bei der Herstellung der 1,2,4-Triazolylderivate entstehen im allgemeinen parallel auch 1,3,4-Triazolylisomere, die sich auf übliche Weise, z. B. mit unterschiedlichen Lösungsmitteln, voneinander trennen lassen.

Die Reaktion (II mit III zu Ia) wird bevorzugt in einem relativ polaren, jedoch reaktionsinerten organischen Lösungsmittel, durchgeführt, z. B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, z. B. Benzol, Toluol, Xylol, Hexan, Petrolether, Chlorbenzol, Nitrobenzol u. a. eingesetzt werden. Die Reaktionstemperaturen liegen in einem Temperaturbereich von 0° bis 150 °C, vorzugsweise 20° bis 100 °C.

Im übrigen kann diese Reaktion (II mit III zu Ia) analog zu bereits bekannten Umsetzungen von anderen Oxiranen mit Azolen [vgl. DE-OS 29 12 288] durchgeführt werden.

Bei den genannten Teilreaktionen können die Zwischenprodukte aus dem Reaktionsmedium isoliert und falls gewünscht, vor der Weiterreaktion, auf eine der allgemein üblichen Methoden gereinigt werden, z. B. durch Waschen, Digerieren, Extraktion, Kristallisation, Chromatographie, Destillation usw.

Die Weiterreaktion von Ia zu I erfolgt in den Fällen, in denen W in Formel IV für eine übliche Abgangsgruppe steht in Abwesenheit oder bevorzugt in Anwesenheit eines reaktionsinerten Lösungsmittels. Es eignen sich z. B. folgende Lösungsmittel : N,N-Dimethylformamid, N,N-Dimethylacetamid, Hexamethylphosphortriamid, Dimethylsulfoxid, 2-Methyl-3-pentanon, usw. Auch Gemische dieser Lösungsmittel untereinander oder mit anderen üblichen inerten organischen Lösungsmitteln, z. B. mit aromatischen Kohlenwasserstoffen wie Benzol, Toluol, Xylolen usw. können verwendet werden. In manchen Fällen kann es sich als vorteilhaft erweisen, zur Beschleunigung der Reaktionsgeschwindigkeit in Gegenwart einer Base wie z. B. eines Alkalimetallhydrides, -hydroxides oder -carbonates zu arbeiten. Es kann aber auch von Vorteil sein, dass man den Alkohol der Formel Ia ($R_3$ = OH) zuerst auf an sich bekannte Weise, z. B. durch Reaktion mit einer starken Base, in ein geeignetes Metallsalz überführt.

Geeignete starke Basen sind z. B. Alkali- und Erdalkalihydride (NaH, KH, $CaH_2$ usw.) und Alkaliorganische Verbindungen wie z. B. Butyllithium oder Alkali-tert.-Butoxid, darüberhinaus können auch Alkalihydroxide, wie NaOH oder KOH eingesetzt werden, wenn man in einem wässrigen Zweiphasensystem und in Anwesenheit eines Phasentransferkatalysators arbeitet.

Man kann jedoch auch den Alkohol der Formel Ia, vor der Weiterreaktion zuerst auf übliche Weise in ein Alkalialkoholat überführen und dann mit einer Verbindung der Formel IV (worin W für eine Abgangsgruppe steht) umsetzen, dabei arbeitet man vorteilhafterweise in Gegenwart eines Kronenethers. Bei M = K, insbesondere in Gegenwart von 18 Krone-6 ; bei M = Na, insbesondere in Gegenwart von 15 Krone-5. Dabei wird die Reaktion zweckmässigerweise in einem reaktionsinerten Medium durchgeführt. Als Lösungsmittel eignen sich z. B. Ether und etherartige Verbindungen, z. B. Diniederalkylether (Diethylether, Diisopropylether, tert.-Butylmethylether usw.), Tetrahydrofuran, Dioxan und aromatische Kohlenwasserstoffe wie Benzol, Toluol oder Xylole.

Für die organische, mit Wasser nicht mischbare Phase kommen dabei z. B. folgende Lösungsmittel in Frage : Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw.. Beispiele geeigneter Phasentransfer-Katalysatoren sind : Tetraalkylammoniumhalogenide, -hydrogensulfate oder -hydroxide wie Tetrabutylammoniumchlorid, -bromid, -jodid ; Triethylbenzylammoniumchlorid, -bromid ; Tetrapropylammoniumchlorid, -bromid, -jodid ; usw.. Als Phasentransfer-Katalysatoren kommen auch Phosphonium-Salze in Betracht. Die Reaktionstemperaturen liegen im allgemeinen zwischen 30° und 130 °C, bzw. am Siedepunkt des Lösungsmittels oder Lösungsmittelgemisches.

In den Fällen, in denen W in Formel IV für Hydroxygruppen stehen, wird vorteilhafterweise eine Kondensationsreaktion durchgeführt. Beide Reaktanden werden in einem geeigneten Lösungenmittel unter Rückfluss erhitzt.

Hierbei können grundsätzlich Lösungsmittel eingesetzt werden, die sich gegenüber den Reaktionspartnern inert verhalten und zweckmässigerweise mit Wasser Azeotrope bilden. Es eignen sich hierzu beispielsweise aromatische Kohlenwasserstoffe, wie Benzol, Toluol, Xylole oder halogenierte Kohlenwasserstoffe, wie Dichlormethan, Chloroform, Tetrachlorkohlenstoff, 1,2-Dichloräthan, Tetrachloräthylen, Chlorbenzol, aber auch ätherartige Verbindungen, wie tert.-Butylmethyläther, Dioxan und andere. In manchen Fällen kann die Verbindung der Formel III selbst als Lösungsmittel verwendet werden. Bei dieser Kondensationsreaktion arbeitet man zweckmässigerweise in Gegenwart einer starken Säure, z. B. Paratoluolsulfonsäure und bei Siedetemperaturen der azeotropen Mischung.

Zur Herstellung der Ether der Formel I kann man auch die freie OH-Gruppe in den Verbindungen der Formel Ia, zuerst gegen eine der obengenannten, üblichen Abgangsgruppen W austauschen und

anschliessend mit einer Verbindung der Formel IV (mit W = OH) umsetzen.

Der Austausch der freien Hydroxylgruppe in den Verbindungen der Formel Ia gegen eine Abgangsgruppe W wird bevorzugt in einem reaktionsinerten Lösungsmittel durchgeführt. Beispiele solcher Lösungsmittel sind : Aromatische und aliphatische Kohlenwasserstoffe wie Benzol, Toluol, Xylole, Petrolether, Ligroin oder Cyclohexan ; halogenierte Kohlenwasserstoffe wie Chlorbenzol, Methylenchlorid, Ethylenchlorid, Chloroform, Tetrachlorkohlenstoff oder Tetrachlorethylen ; Ether und etherartige Verbindungen wie Diethylether, Diisopropylether, t-Butylmethylether, Dimethoxyethan, Dioxan, Tetrahydrofuran oder Anisol ; Ester wie Ethylacetat, Propylacetat oder Butylacetat ; Nitrile wie Acetonitril oder Verbindungen wie Dimethylsulfoxid, Dimethylformamid und Gemische solcher Lösungsmittel untereinander.

Die Einführung der Abgangsgruppe W erfolgt nach üblichen Methoden. Bedeutet A Chlor, so wird als Reagenz z. B. Phosphoroxychlorid, Phosphortrichlorid, Phosphorpentachlorid oder vorzugsweise Thionylchlorid eingesetzt. Man arbeitet im allgemeinen bei Temperaturen von 0° bis + 120 °C. Im Falle von W = Brom verwendet man bevorzugt Phosphortribromid oder Phosphorpentabromid und führt die Reaktion bei 0° bis + 50 °C durch. Steht W für eine der Gruppen $—OSO_2R_a$, $—OCO—R_a$ oder

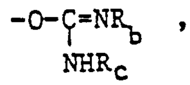

so wird als Reagenz üblicherweise das entsprechende Säurechlorid bzw. Amidinochlorid eingesetzt. Hierbei ist es zweckmässig, wenn die Reaktion bei Temperaturen von — 20° bis + 50 °C, vorzugsweise — 10° bis + 30 °C, und in Gegenwart einer schwachen Base wie Pyridin oder Triethylamin durchgeführt wird.

Die Ausgangsprodukte der Formeln III und IV sind allgemein bekannt oder lassen sich nach an sich bekannten Methoden herstellen.

Die Oxirane der Formel II sind neu, sie stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindungen der Formel I überführen, darüberhinaus zeigen Verbindungen der Formel II zum Teil fungizide Aktivität gegenüber Schadpilzen aus den Familien Ascomycetes, Basidiomycetes oder Fungi imperfecti.

Die Oxirane der Formel II lassen sich in an sich bekannter Weise durch Reaktion der zugrundeliegenden Ketone der Formel V

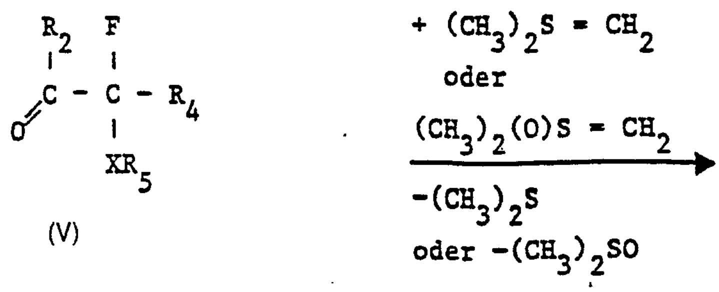


(V) (II)

mit Dimethylsulfoniummethylid, Dimethyloxosulfoniummethylid oder mit einem ihrer Salze, wie z. B. dem Methyljodid, in Dimethylsulfoxid oder einem anderen bei der Reaktion II mit III beschriebenen Lösungsmittel herstellen. In Formel V haben die Substituenten die unter Formel I angegebenen Bedeutungen. Die Reaktion wird bei Temperaturen von 0° bis 120 °C durchgeführt.

Analoge Reaktionen sind aus der Literatur bekannt ; vgl. JACS, 87, 1353 (1965). Man kann die Reaktion prinzipiell analog zu den dort beschriebenen Umsetzungen vornehmen. Ketone der Formel V können z. B. aus den an sich bekannten α-Fluor-α-halogenketonen der Formel VI

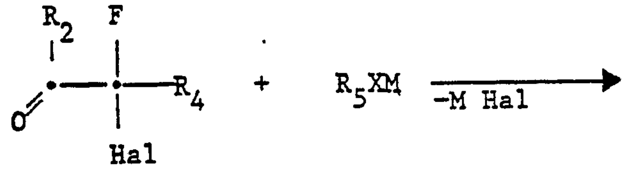


(V)

(VI) (VII)

mit Verbindungen der Formel VII in üblichen reaktionsinerten Lösungsmitteln und gegebenenfalls bei erhöhter Temperatur hergestellt werden. In den Formeln VI und VII haben die Substituenten $R_2$, $R_3$, $R_4$, $R_5$ und X die unter Formel I angegebenen Bedeutungen, Hal steht für Halogen, vorzugsweise für Chlor oder Brom und M bedeutet vorzugsweise ein Metallatom, insbesondere Natrium oder Kalium.

Die Verbindungen der Formel VII sind bekannt oder können nach an sich bekannten Verfahren hergestellt werden.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether ; halogenierte Kohlenwasserstoffe wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril ; N,N-dialkylierte Amide wie Dimethylformamid ; Dimethylsulfoxid ; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon oder in gewissen Fällen auch Kohlendioxid.

Das beschriebene Herstellungsverfahren ist, einschliesslich aller Teilschritte, ein wichtiger Bestandteil der vorliegenden Erfindung.

Die Verbindungen der Formel I

$$R_1 - CH_2 - \overset{\overset{R_2}{|}}{\underset{\underset{OR_3}{|}}{C^*}} - \overset{\overset{F}{|}}{\underset{\underset{XR_5}{|}}{C^*}} - R_4 \qquad (I)$$

besitzen in Nachbarstellung zu jedem der Substituenten $OR_3$ und $XR_5$ je ein asymmetrisches C-Atom C* und können daher in enantiomeren Formen vorliegen. Im allgemeinen entsteht bei der Herstellung dieser Substanzen, wenn $R_4 = F$ ist, ein Gemisch von zwei Diastereomeren. Dieses lässt sich nach physikalischen Methoden (z. B. durch Kristallisation, Chromatographie oder Destillation) voneinander trennen. Die zugrundeliegenden Enantiomere können auf übliche Weise, z. B. durch fraktionierte Kristallisation von Salzen mit optisch aktiven, starken Säuren, in die reinen optischen Antipoden aufspalten. Die Enantiomeren oder Diastereomeren können unterschiedliche biologische Wirkungen aufweisen, so kann z. B. bei der einen Form die fungizide und bei der anderen die pflanzenregulatorische Wirkung im Vordergrund stehen. Auch kann bei gleichem Wirkungsspektrum ein gradueller Aktivitätsunterschied auftreten.

Die vorliegende Erfindung betrifft alle reinen Stereoisomeren, Enantiomeren und deren Gemisch untereinander.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hangen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegativen Pflanzenwachstums eingesetzt werden. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstrums bei Getreide, denn durch eine Halmverkürzung wird die Gefahr des Umkickens (« Lagerns ») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen

Wachstums führen, so dass z. B. mehr oder grössere Früchte zur Ausbildung kommen.

Ertragssteigerungen können in manchen Fällen auch durch einen Eingriff in den pflanzlichen Stoffwechsel erreicht werden, ohne dass sich Aenderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von Pflanzen. Es ist jedoch auch möglich, das Wachstum der Seitentriebe zu hemmen, z. B. um bei Tabakpflanzen nach der Dekapitierung die Ausbildung von Seitentrieben zu verhindern und damit das Blattwachstum zu fördern.

Unter dem Einfluss von Wachstumsregulatoren kann der Blattbestand von Pflanzen so gesteuert werden, dass ein Entblättern der Pflanzen zu einem gewünschten Zeitpunkt erreicht wird, Eine derartige Entlaubung ist von Interesse, um eine mechanische Beerntung z. B. bei Wein oder Baumwolle, zu erleichtern oder um die Transpiration zu einem Zeitpunkt herabzusetzen, an dem die Pflanze verpflanzt werden soll. Durch Einsatz von Wachstumsregulatoren lässt sich der vorzeitige Fruchtfall verhindern. Es ist jedoch auch möglich, den Fruchtfall, — z. B. bei Obst —, im Sinne einer chemischen Ausdünnung bis zu einem bestimmten Ausmass zu fördern. Wachstumsregulatoren können auch dazu dienen, um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Voraussetzungen dafür geschaffen, dass z. B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen, also die endogene Jahresrhythmik, beeinflusst werden, so dass die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z. B. um in frostgefährdeten Gebieten eine Schädigung durch Spätfröste zu vermeiden. Andererseits gelingt es, das Wurzelwachstum zu fördern und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Cover crops. In tropischen und subtropischen Monokulturen, wie z. B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungsplanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw;

entsprechende Mittel, ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam : Ascomyceten (z. B. Venturia, Podosphaera, Erysiphe, Monilinia, Uncinula) ; Basidiomyceten (z. B. die Gattungen Hemileia, Rhizoctonia, Puccinia) ; Fungi imperfecti (z. B. Botrytis, Helminthosporium, Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehrere hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten : Getreide : (Weizen, Gerste, Roggen, Hafer, Reis, Sorghum und Verwandte) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) ; oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden im Agrarbereich üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln. Vorteilhafte Formulierungshilfsstoffe sind ferner Phospholipide.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwert (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen,

verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechen gewählt. Günstige Aufwandmengen liegen im allgemeinen bei 10 g bis 5 kg Aktivsubstanz (AS) je ha ; bevorzugt 100 g bis 2 kg AS/ha, insbesondere bei 200 g bis 600 g AS/ha.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Staübemittel und dispergierbare Pulver, werden in der Regel natürliche Gesteinsmehele verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bisstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$) genannt, wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8-22 C-Atomen. Alkylarylsulfonate sind z. B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Ethylenoxyd-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatomen im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 150 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die

Salze liegen vorzugsweise als Halogenide, Methylsulfate oder Ethylsulfate vor, z. B. das Stearyltrimethylammoniumchlorid oder das Benzyldi(2-chlorethyl)-ethylammoniumbromid.

Die in der Formulierungstechnik gebräuchlichen Tenside sind u. a. in folgenden Publikationen beschrieben : « Mc Cutcheon's Detergents and Emulsifiers Annual » MC Publishing Corp., Ridgewood, New Jersey, 1981 ; H. Stache, « Tensid-Taschenbuch », 2. Aufl., C. Hanser Verlag, München, Wien, 1981 ; M. and J. Ash. « Encyclopedia of Surfactants », Vol. I-III, Chemical Publishing Co., New York, 1980-1981.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes darunter 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben. Prozente und Teile beziehen sich auf das Gewicht. RT bedeutet Raumtemperatur, h steht für Stunde, min für Minute, DMSO für Dimethylsulfoxid, THF für Tetrahydrofuran, DMF für Dimethylformamid.

Beispiel H1

a) Herstellung der Vorstufe

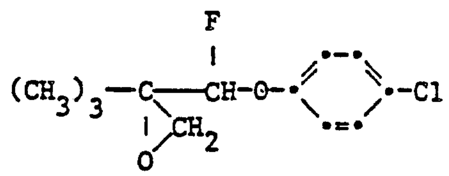

2-tert.-Butyl-2-[(4-chlorphenoxy)-fluormethyl]-oxiran

Zu einer Dispersion von 5,28 g 80 %igem Natriumhydrid in 300 ml absolutem DMSO wurden unter Stickstoffatmosphäre portionsweise unter Rühren 43 g Trimethyloxosulfoniumjodid gegeben. Nach Abklingen der exothermen Reaktion wurde das Gemisch noch 1 h gerührt und anschliessend bei RT eine Lösung von 40 g 1-(4-Chlorphenoxy)-3,3-dimethyl-1-fluor-2-butanon in 100 ml THF zugetropft und das Gemisch weitere 2 h bei RT gerührt. Anschliessend wurde die Reaktionsmischung auf Eiswasser gegossen und mehrfach mit Diethylether extrahiert. Die vereinigten Extrakte wurden mit Wasser gewaschen, über Natriumsulfat getrocknet, filtriert und eingeengt. Ausbeute des Rohprodukts 41,5 g, als gelbbraunes Oel, das entweder säulenchromatographisch gereinigt oder ungereinigt weiterverarbeitet werden kann.

b) Herstellung des Endproduktes

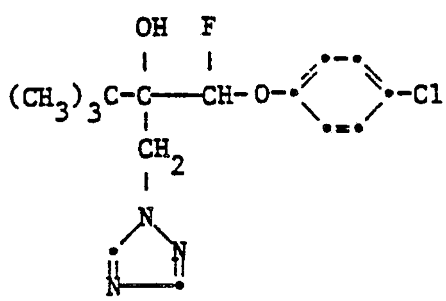

(Verb. Nr. 1.1)

1-Fluor-1-(4-chlorphenoxy)-2-(1H-1,2,4-triazol-1'-ylmethyl)-3,3-dimethyl-butan-2-ol

20,9 g des nach a) hergestellten 2-tert.-Butyl-2-[(4-chlorphenoxy)-fluormethyl]-oxirans wurden in 300 ml DMF gelöst und mit 8,3 g 1,2,4-Triazol und 0,9 g Kalium-tert.-butanolat versetzt. Das Reaktionsgemisch wurde 5 h gerührt und dabei auf 120 °C erwärmt, anschliessend auf RT abgekühlt, mit dem dreifachen Volumen Eiswasser verdünnt und mehrfach mit Diethylether extrahiert. Die vereinigten Extrakte wurden mit Wasser gewaschen, über Natriumsulfat getrocknet, filtriert und eingeengt. Ausbeute 23 g, in Form eines farblosen Oels.

Auf Analoge Weise lassen sich auch die nachfolgenden Produkte herstellen :

(Siehe Tabellen Seite 11 ff.)

Tabelle 1

Verbindungen der Formel 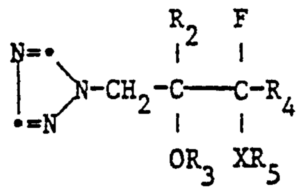

| Verb. Nr. | $R_2$ | $R_3$ | $R_4$ | $R_5$ | X | Physik. Konst. |
|---|---|---|---|---|---|---|
| 1.1 | t-Butyl | H | H | $C_6H_4Cl(4)$ | O | $n_D^{50}$ 1.5223 |
| 1.2 | t-Butyl | H | H | $C_6H_4F(4)$ | O | $n_D^{50}$ 1.5041 |
| 1.3 | t-Butyl | H | H | $C_6H_4Br(4)$ | O | Smp.124-125°C |
| 1.4 | t-Butyl | H | H | $C_6H_4CH_3(4)$ | O | Smp.110-111°C |
| 1.5 | t-Butyl | H | H | $C_6H_4OCH_3(4)$ | O | Smp. 94-95°C |
| 1.6 | t-Butyl | H | H | $C_6H_4OCF_3(4)$ | O | Smp.117-118°C |
| 1.7 | t-Butyl | H | $CH_3$ | $C_6H_4F(4)$ | O | |
| 1.8 | t-Butyl | H | $C_2H_5$ | $C_6H_4F(4)$ | O | |
| 1.9 | t-Butyl | H | F | $C_6H_4Cl(4)$ | O | |
| 1.10 | t-Butyl | H | H | $C_6H_4OCHF_2(4)$ | O | |
| 1.11 | t-Butyl | H | H | $C_6H_3Cl_2(2,4)$ | O | visk. Oel |
| 1.12 | t-Butyl | H | $CH_3$ | $C_6H_4Br(4)$ | O | |
| 1.13 | t-Butyl | H | H | $C_6H_4Cl(4)$ | S | |
| 1.14 | t-Butyl | H | H | $C_6H_4F(4)$ | S | |
| 1.15 | t-Butyl | H | H | $C_6H_4CF_3(4)$ | O | Smp.125-127°C |
| 1.16 | t-Butyl | H | H | $C_6H_3Cl(2,3)$ | O | Smp.115-120°C |
| 1.17 | t-Butyl | $CH_3$ | H | $C_6H_4F(4)$ | O | |
| 1.18 | t-Butyl | $CH_3$ | H | $C_6H_4Cl(4)$ | O | |
| 1.19 | t-Butyl | $CH_3$ | H | $C_6H_4Br(4)$ | O | |
| 1.20 | t-Butyl | $CH_3$ | H | $C_6H_4OCF_3(4)$ | O | |
| 1.21 | t-Butyl | Benzyl | H | $C_6H_4CH_3(4)$ | O | |
| 1.22 | t-Butyl | Benzyl | H | $C_6H_4Cl(4)$ | O | |
| 1.23 | t-Butyl | Benzyl | H | $C_6H_4CH_3(4)$ | O | |
| 1.24 | i-Propyl | H | H | $C_6H_4F(4)$ | O | |

Tabelle 1 (Fortsetzung)

| Verb. Nr. | $R_2$ | $R_3$ | $R_4$ | $R_5$ | X | Physik. Konst. |
|---|---|---|---|---|---|---|
| 1.25 | i-Propyl | H | H | $C_6H_4Cl(4)$ | O | |
| 1.26 | i-Propyl | H | H | $C_6H_5$ | O | |
| 1.27 | t-Butyl | H | H | $C_6H_5$ | O | |
| 1.28 | t-Butyl | $CH_3$ | H | $C_6H_5$ | O | |
| 1.29 | t-Butyl | $CH_3$ | $CH_3$ | $C_6H_4F(4)$ | O | |
| 1.30 | t-Butyl | H | H | α-Naphthyl | O | |
| 1.31 | t-Butyl | H | H | $C_6H_4-C_6H_5(4)$ | O | |
| 1.32 | t-Butyl | H | H | $C_6H_4Cl(4)$ | O | Smp. 119-120° |
| 1.33 | t-Butyl | H | H | $C_6H_3(CH_3)_2(2,3)$ | O | Harz |

Tabelle 2

Verbindungen der Formel 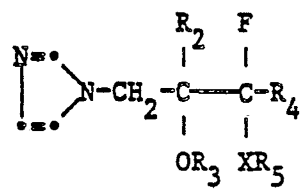

| Verb. Nr. | $R_2$ | $R_3$ | $R_4$ | $R_5$ | X | Physik. Konst. |
|---|---|---|---|---|---|---|
| 2.1 | t-Butyl | H | H | $C_6H_4Cl(4)$ | O | $n_D^{50}$ 1.5311 |
| 2.2 | t-Butyl | H | H | $C_6H_4F(4)$ | O | |
| 2.3 | t-Butyl | H | H | $C_6H_5$ | O | |
| 2.4 | t-Butyl | H | H | $C_6H_4Br(4)$ | O | |
| 2.5 | t-Butyl | H | H | $C_6H_4CH_3(4)$ | O | |
| 2.6 | t-Butyl | H | H | $C_6H_4Cl(2)$ | O | |
| 2.7 | t-Butyl | H | H | $C_6H_4CF_3(4)$ | O | |
| 2.8 | t-Butyl | H | H | $C_6H_4OCF_3(4)$ | O | |
| 2.9 | t-Butyl | H | H | $C_6H_4OCF_2H(4)$ | O | |
| 2.10 | i-Propyl | H | H | $C_6H_5$ | O | |
| 2.11 | i-Propyl | H | H | $C_6H_4Cl(4)$ | O | |
| 2.12 | i-Propyl | H | H | $C_6H_4F(4)$ | O | |
| 2.13 | t-Butyl | H | F | $C_6H_4Cl(4)$ | O | $n_D^{50}$ 1.5136 |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenol-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen von Trägerstoffen mit dem Wirkstoff erhält man gebrauchsfertige Stäubemittel.

Formulierungsbeispiele für feste Wirkstoffe der Formel I (% = Gewichtsprozent)

F5. Spritzpulver

| | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

F7. Stäubemittel

| | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

F8. Extruder-Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3 % |
| Polyethylenglykol (M G 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Lingninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele :

Beispiel B1

Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,002 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpusteinentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006 %) Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen 1 und 2 zeigten gegen Puccinia-Pilze eine sehr gute Wirkung. Unbehandelte aber infizierte Kontrollpflanzen zeigten einen Puccinia-Befall von 100 %. Unter anderem hemmten die Verbindungen 1.1, 1.2, 1.11, und 2.1 den Puccinia-Befall auf 0 bis 5 %.

Beispiel B2

Wirkung gegen Cercospora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhinderten die Verbindungen 1.1, 1.2 und 2.1 in obigen Versuchen das Auftreten von Flecken fast vollständig (0-10 %).

Beispiel B3

Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,002 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,000 6 % Aktivsubstanz bezogen auf das Erdvolumen). Es wurde dabei darauf

geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100 %. Unter anderen Verbindungen aus den Tabellen 1 und 2 hemmten die Verbindungen Nr. 1.1, 1.2, 1.11 und 2.1 den Pilzbefall bei Gerste auf 0 bis 5 %.

## Beispiel B4

Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,006 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Verbindungen 1.1, 1.2, 2.1 und andere hemmten den Krankheitsbefall auf weniger als 20 %. Unbehandelte aber infizierte Triebe zeigten einen 100 %igen Venturia-Befall.

## Beispiel B5

Wirkung gegen Botrytis cinerea auf Aepfeln

Residual protektive Wirkung

Künstlich verletzte Aepfel wurden behandelt, indem eine aus Spritzpulver der Wirksubstanz hergestellte Spritzbrühe (0,02 % Aktivsubstanz) auf die Verletzungsstellen aufgetropft wurde. Die behandelten Früchte wurden anschliessend mit einer Sporensuspension von Botrytis cinerea inokuliert und während einer Woche bei hoher Luftfeuchtigkeit und ca. 20 °C inkubiert.

Das Vorhandensein und die Grösse der Fäulnis-Stellen an der Frucht dienten zur Bewertung der fungiziden Aktivität. Bei Behandlung mit Verbindungen aus den Tabellen 1 und 2, z. B. Nr. 1.1, 1.2 und 2.1 wurden keine oder fast keine Fäulnis-Stellen (0-5 % Befall) beobachtet.

## Beispiel B6

Wuchshemmung bei Getreide

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0.3, 0.5, 1.0, 2.5 bzw. 3.0 kg Aktivsubstanz pro Hektar. 21 Tage nach Applikation wurde das Wachstum des Getreides beurteilt. Hierbei konnte festgestellt werden, dass Getreidepflanzen, die mit Wirkstoffen der Formel I behandelt worden waren, im Vergleich zu unbehandelten Kontrollpflanzen eine starke Wuchsreduktion aufweisen. Als besonders wirksam erwiesen sich Verbindungen aus den Tabellen 1 und 2.

Testresultate : Wuchshöhe der Getreidepflanzen in Prozent zur Wuchshöhe von unbehandelten Kontrollpflanzen.

| Verb. Nr. | 3 kgAS/ha | | 2.5 kgAS/ha | | 1.0 kgAS/ha | | 0.5 kgAS/ha | | 0.3 kgAS/ha | |
|---|---|---|---|---|---|---|---|---|---|---|
| | Roggen | Gerste | Roggen | Gerste | Roggen | Gerste | Roggen | Gerste | Roggen | Gerste |
| 1.1 | - | - | 39 | 11 | - | - | 61 | 32 | - | - |
| 1.2 | - | - | 20 | 16 | - | - | 20 | 33 | - | - |
| 1.11 | 65 | 54 | - | - | 84 | 85 | - | - | 88 | 87 |
| 2.1 | - | - | 83 | 74 | - | - | 94 | 100 | - | - |

Beispiel B7

Wuchshemmung bei Gräsern

In Kunststoffschalen mit Erde-Torf-Sand-Gemisch (6:3:1) wurden die Gräser Lolium perenne, Poa pratensis, Festuca orina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 0.3, 0.5, 1.0, 2.5 bzw. 3.0 kg Aktivsubstanz pro Hektar. 31 Tage nach Applikation wurde das Wachstum der Gräser beurteilt, dabei zeigte es sich, dass die erfindungsgemässen Wirkstoffe aus den Tabellen 1 und 2 eine merkliche Wuchshemmung bewirkten.

Testresultate : Wuchshöhe von Gräsern in Prozent zur Wuchshöhe der unbehandelten Kontrollpflanzen.

| Verb. Nr. | 3 kgAS/ha | 2.5 kgAS/ha | 1.0 kgAS/ha | 0.5 kgAS/ha | 0.3 kgAS/ha |
|---|---|---|---|---|---|
| 1.1 | - | 31 | - | 56 | - |
| 1.2 | - | 26 | - | 31 | - |
| 1.11 | 52 | - | 56 | - | 92 |
| 2.1 | - | 54 | - | 94 | - |

Beispiel B8

Ertragssteigerung an Sojabohnen

In Kunststoffbehältern mit einem Erde-Torf-Sandgemisch im Verhältnis 6 : 3 : 1 wurden Sojabohnen der Sorte « Hark » angesät und in eine Klimakammer gegeben. Durch optimale Temperaturwahl, Beleuchtung, Düngerzugabe und Bewässerung entwickelten sich die Pflanzen nach ca. 5 Wochen in dem 5-6 Trifolia-Blattstadium. Zu diesem Zeitpunkt wurden die Pflanzen mit der wässrigen Brühe eines Wirkstoffes der Formel I bis zur guten Benetzung besprüht. Die Wirkstoffkonzentration betrug bis zu 500 ppm Aktivsubstanz. Die Auswertung erfolgte ca. 5 Wochen nach der Applikation des Wirkstoffs. Im Vergleich zu unbehandelten Kontrollpflanzen bewirkten die erfindungsgemässen Wirkstoffe der Formel I eine merkliche Erhöhung der Anzahl und des Gewichtes der geernteten Schoten. Als besonders wirksam erwiesen sich die Verbindungen aus den Tabellen 1 und 2.

Beispiel B9

Wuchshemmung bei Bodenbedecker-Pflanzen (Cover-Crops)

In Kunststoffschalen mit Erde-Torf-Sand-Gemisch (1 : 1 : 1) werden Testpflanzen der Sorte Centrosema plumieri und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27 °C und einer Nachttemperatur von 21 °C, bei einer mittleren Lichtdauer von 14 h (6 000 Lux) und einer Luftfeuchtigkeit von 70 % statt. Die Testpflanzen werden auf eine Höhe von ca. 15 cm zurückgeschnitten und 7 Tage nach dem Zurückschneiden mit einer Spritzbrühe des Wirkstoffes (umgerechnet 0.3, 1.0 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus der Tabelle 1 eine deutliche Wuchshemmung der Bodenbedecker-Pflanzen auslösen.

Testresultate : Wuchshöhe von cover-crop-Pflanzen in Prozent zur Wuchshöhe der unbehandelten Kontrollpflanzen.

| Verb.Nr. | Centrosema pubescens | | | Psophocarpus palustris | | |
|---|---|---|---|---|---|---|
| | kgAS/ha | | | kgAS/ha | | |
| | 3.0 | 1.0 | 0.3 | 3.0 | 1.0 | 0.3 |
| 1.1 | 10 | 20 | 10 | 10 | 10 | 30 |
| 1.2 | 10 | 10 | 10 | 10 | 10 | 40 |
| 1.11 | 10 | 20 | 50 | 10 | 40 | 60 |

Beispiel B10

Seneszenzhemmung bei Getreidepflanzen

Im Gewächshaus wird Sommerweisen der Sorte « Svenno » in Töpfen mit Komposterde angesät und ohne spezielle klimatische Anforderungen gezogen. Ca. 10 Tage nach dem Auflaufen werden 10 bis 12 cm lange Primärblätter abgeschnitten und einzeln in Reagenzgläser mit 10 ml einer Wirkstoffsuspension (1,25 bis 10 ppm Aktivsubstanz) gegeben. Die Reagenzgläser werden in einem klimatisierten Raum bei 23 °C, 70 % relativer Luftfeuchtigkeit aufgestellt und täglich durchschnittlich 14 Stunden (10 000 Lux) bestrahlt. Die Beurteilung der Seneszenzhemmung erfolgt 7 Tage nach Einstellen der Blätter durch Vergleich des Vergilbungsgrades im Verhältnis zu noch frischen, grünen Blättern. Bei diesem Versuch kann beobachtet werden, dass Verbindungen aus den Tabellen 1 und 2 eine deutliche Inhibierung der Seneszenz der Testpflanzen hervorrufen.

Beispiel B11

Hemmung des Vegetativ-Wachstums an Soja

In Kunststofftöpfen mit einem Erde-Torf-Sandgemisch im Verhältnis 6 : 3 : 1 werden die Sojabohnen der Sorte « Hark » angesät, im Gewächshaus aufgestellt und nach Bedarf bewässert. 15 Tage nach der Saat werden die Pflanzen mit der wässrigen Spritzbrühe eines Wirkstoffes der Formel I bis zur Benetzung besprüht. Die Wirkstoffkombination beträgt umgerechnet 0.1, 0.5 oder 1.5 kg Aktivsubstanz pro Hektar. 14 Tage nach der Applikation wird das Wachstum der Pflanzen beurteilt. Dabei zeigt sich das die erfindungsgemässen Wirkstoffe aus den Tabellen 1 und 2 eine merkliche Wuchshemmung bewirken.

Testresultate : Wuchshöhe von Sojapflanzen in Prozent zur Wuchshöhe von unbehandelten Kontrollpflanzen.

| Verb. Nr. | 1.5 kgAS/ha | 0.5 kgAS/ha | 0.1 kgAs/ha |
|---|---|---|---|
| 1.1 | 8 | 8 | 16 |
| 1.2 | 9 | 14 | 68 |
| 1.11 | 18 | 24 | 24 |
| 2.1 | 79 | 83 | 92 |

**Patentansprüche** (für die Vertragsstaaten : BE, CH, DE, FR, GB, IT, LI, NL, SE)

1. Verbindungen der Formel

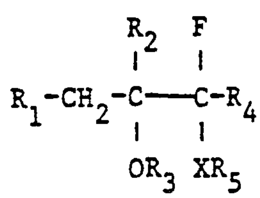

(I)

worin $R_1$ für eine 1,2,4-Triazolyl- oder Imidazolylgruppe steht ; $R_2$ $C_1$-$C_{12}$-Alkyl bedeutet ; $R_3$ Wasserstoff, $C_1$-$C_{12}$-Alkyl, $C_2$-$C_4$-Alkenyl, $C_2$-$C_4$-Alkinyl oder gegebenenfalls durch $C_1$-$C_6$-Alkyl, $C_1$-$C_6$-Alkoxy, $C_1$-$C_6$-Haloalkyl, Halogen und/oder Cyano substituiertes Benzyl bedeutet ; $R_4$ für Wasserstoff, Fluor oder $C_1$-$C_6$-Alkyl steht ; $R_5$ für einen unsubstituierten oder substituierten Rest, ausgewählt aus der Reihe Phenyl, Naphthyl, Biphenyl, Benzylphenyl und Benzyloxyphenyl steht, wobei die Substituenten aus der Reihe Halogen, Cyano, $C_1$-$C_6$-Alkyl, $C_1$-$C_6$-Alkoxy, $C_1$-$C_6$-Haloalkyl, $C_1$-$C_6$-Alkylthio, $C_1$-$C_6$-Haloalkoxy, $C_1$-$C_3$-Haloaklylthio, Nitro und/oder Rhodano ausgewählt sind ; und X Sauerstoff oder Schwefel bedeutet ; unter Einschluss der Säureadditionssalze, quaternären Azoliumsalze und Metallkomplexe.

2. Verbindungen der Formel I gemäss Anspruch 1, worin $R_2$ $C_1$-$C_6$-Alkyl bedeutet ; $R_3$ Wasserstoff, $C_1$-$C_6$-Alkyl, Allyl, Propargyl, Benzyl, 2,6-Dichlorbenzyl, 4-Chlorbenzyl, 4-Fluorbenzyl, 2,4-Dichlorbenzyl oder 2-Chlor-4-fluorbenzyl beudet ; $R_4$ für Wasserstoff oder Methyl steht, $R_5$ Phenyl, 4-Halophenyl, 2,4-Dihalophenyl oder durch $CF_3$ substituiertes Phenyl steht und X Sauerstoff oder Schwefel bedeutet.

3. Verbindungen der Formel I gemäss Anspruch 2, worin $R_1$ für 1,2,4-Triazol steht ; $R_2$ für tert.-Butyl oder iso-Propyl steht ; $R_3$ Wasserstoff oder $C_1$-$C_5$-Alkyl bedeutet ; $R_4$ Wasserstoff oder Methyl bedeutet ; $R_5$ für 4-Halophenyl, 2,4-Dihalophenyl oder $4—CF_3—C_6—H_4—$ steht und X Sauerstoff bedeutet.

4. Verbindungen der Formel I gemäss Anspruch 3, worin $R_3$ für Wasserstoff steht und die übrigen Substituenten wie in Anspruch 3 definiert sind.

5. Eine Verbindung der Formel I gemäss Anspruch 1, ausgewählt aus der Reihe : 1-Fluor-1-(4-chlorphenoxy)-2-(1H-1,2,4-triazol-1'-ylmethyl)-3,3-dimethyl-butan-2-ol, 1-Fluor-1-(4-fluorphenoxy)-2-(1H-1,2,4-triazol-1'-ylmethyl)-3,3-dimethyl-butan-2-ol, 1-Fluor-1-(4-chlorphenoxy)-2-(1H-imidazol-1'-ylmethyl)-3,3-dimethyl-butan-2-ol, 1-Fluor-1-(2,4-dichlorphenoxy)-2-(1H-1,2,4-triazol-1'-ylmethyl)-3,3-dimethylbutan-2-ol.

6. Verfahren zur Herstellung der in Anspruch 1 definierten Verbindungen der Formel I, dadurch gekennzeichnet, dass man ein Oxiran der Formel II

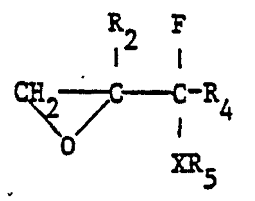

(II)

mit einem Azol der Formel III

$$M—R_1 \qquad (III)$$

zuerst zu einem Alkohol der Formel Ia

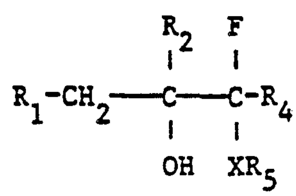

(Ia)

umsetzt und den Alkohol der Formel Ia, sofern ein Ether der Formel I hergestellt werden soll, auf übliche Art, z. B. durch Reaktion mit einer Verbindung der Formel IV

$$R_3—W \qquad (IV)$$

in einen Ether der Formel I überführt, wobei die Substituenten $R_1$ bis $R_5$ in den Formeln Ia, II, III und IV die unter Formel I angegebenen Bedeutungen haben, M für Wasserstoff oder ein Metallatom steht, Hal Halogen bedeutet, und W für OH oder eine übliche Abgangsgruppe steht.

7. Mittel zur Bekämpfung oder Verhütung eines Befalls durch Mikroorganismen und/oder zur Regulierung des Pflanzenwachstums, dadurch gekennzeichnet, dass es als mindestens eine aktive Komponente eine Verbindung der Formel I gemäss Anspruch 1 enthält.

8. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I auf die Pflanze oder deren Standort appliziert.

9. Verwendung von Verbindungen der Formel I gemäss Anspruch 1 zur Bekämpfung und/oder zur präventiven Verhütung eines Befalls von pflanzenschädlichen Mikroorganismen und/oder zur Regulierung des Pflanzenwachstums.

10. Verwendung gemäss Anspruch 9, dadurch gekennzeichnet, dass es sich bei den Mikroorganismen um phytopathogene Pilze handelt.

11. Verwendung gemäss Anspruch 10 gegen Pilze aus den Klassen Ascomycetes, Basidiomycetes oder Fungi imperfecti.

12. Verwendung gemäss Anspruch 9 zur Wuchshemmung im Sinne einer Erhöhung der Knickfestigkeit und Halmverkürzung bei Getreidesorten.

13. Verwendung gemäss Anspruch 12, dadurch gekennzeichnet, dass es sich bei den Getreidesorten um Hafer, Weisen, Gerste oder Roggen handelt.

14. Verwendung gemäss Anspruch 9 zur Wuchshemmung bei Gräsern.

15. Verwendung gemäss Anspruch 9 zur Wuchshemmung bei Bodenbedeckerpflanzen.

16. Verwendung gemäss Anspruch 9 zur Wuchsregulierung bei Leguminosen im Sinne einer Ertragssteigerung.

17. Verwendung gemäss Anspruch 16, dadurch gekennzeichnet, dass es sich um Soja handelt.

18. Verwendung gemäss Anspruch 16 zur Ertragssteigerung durch Seneszenzhemmung in Getreide, Soja und Baumwolle.

19. Die Oxirane der Formel II

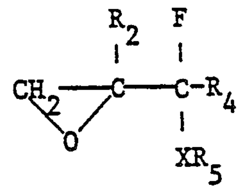

(II)

worin $R_2$, $R_4$ und $R_5$ die in Anspruch 1 unter Formel I angegebenen Bedeutungen haben.

**Patentansprüche** (für den Vertragsstaat AT)

1. Mittel zur Bekämpfung oder Verhütung eines Befalls durch Mikroorganismen und/oder zur Regulierung des Pflanzenwachstums, dadurch gekennzeichnet, dass es als aktive Komponente mindestens eine Verbindung der Formel I

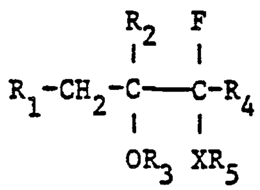

(I)

enthält, worin $R_1$ für eine 1,2,4-Triazolyl- oder Imidazolylgruppe, steht ; $R_2$ $C_1$-$C_{12}$-Alkyl bedeutet ; $R_3$ Wasserstoff, $C_1$-$C_{12}$-Alkyl, $C_2$-$C_4$-Alkenyl, $C_2$-$C_4$-Alkinyl oder gegebenenfalls durch $C_1$-$C_6$-Alkyl, $C_1$-$C_6$-Alkoxy, $C_1$-$C_6$-Haloalkyl, Halogen und/oder Cyano substituiertes Benzyl bedeutet ; $R_4$ für Wasserstoff, Fluor oder $C_1$-$C_6$-Alkyl steht ; $R_5$ für einen unsubstituierten oder substituierten Rest, ausgewählt aus der Reihe Phenyl, Naphthyl, Biphenyl, Benzylphenyl und Benzyloxyphenyl steht, wobei die Substituenten aus der Reihe Halogen, Cyano, $C_1$-$C_6$-Alkyl, $C_1$-$C_6$-Alkoxy, $C_1$-$C_6$-Haloalkyl, $C_1$-$C_6$-Alkylthio, $C_1$-$C_6$-Haloalkoxy, $C_1$-$C_3$-Haloalkylthio, Nitro und/oder Rhodano ausgewählt sind ; und X Sauerstoff oder Schwefel bedeutet ; unter Einschluss der Säureadditionssalze, quaternären Azoliumsalze und Metallkomplexe.

2. Mittel gemäss Anspruch 1, worin $R_2$ $C_1$-$C_6$-Alkyl bedeutet ; $R_3$ Wasserstoff, $C_1$-$C_6$-Alkyl, Allyl, Propargyl, Benzyl, 2,6-Dichlorbenzyl, 4-Chlorbenzyl, 4-Fluorbenzyl, 2,4-Dichlorbenzyl oder 2-Chlor-4-fluorbenzyl bedeutet ; $R_4$ für Wasserstoff oder Methyl steht, $R_5$ Phenyl, 4-Halophenyl, 2,4-Dihalophenyl oder durch $CF_3$ substituiertes Phenyl steht und X Sauerstoff oder Schwefel bedeutet.

3. Mittel gemäss Anspruch 2, worin $R_1$ für 1,2,4-Triazol steht ; $R_2$ für tert.-Butyl oder iso-Propyl steht ; $R_3$ Wasserstoff oder $C_1$-$C_5$-Alkyl bedeutet ; $R_4$ Wasserstoff oder Methyl bedeutet ; $R_5$ für 4-Halophenyl, 2,4-Dihalophenyl oder $4—CF_3—C_6H_4—$ steht und X Sauerstoff bedeutet.

4. Mittel gemäss Anspruch 3, worin $R_3$ für Wasserstoff steht und die übrigen Substituenten wie in Anspruch 3 definiert sind.

5. Mittel gemäss Anspruch 1, dadurch gekennzeichnet, dass es einen Wirkstoff aus der Reihe 1-Fluor-1-(4-chlorphenoxy)-2-(1H-1,2,4-triazol-1'-ylmethyl)-3,3-dimethyl-butan-2-ol, 1-Fluor-1-(4-fluorphenoxy)-2-(1H-1,2,4-triazol-1'-ylmethyl)-3,3-dimethyl-butan-2-ol, 1-Fluor-1-(4-chlorphenoxy)-2-(1H-imidazol-1'-ylmethyl)-3,3-dimethylbutan-2-ol oder 1-Fluor-1-(2,4-dichlorphenoxy)-2-(1H-1,2,4-triazol-1'-ylmethyl)-3,3-dimethylbutan-2-ol enthält.

6. Verfahren zur Herstellung der Verbindungen der Formel 1, gemäss Anspruch 1, dadurch gekennzeichnet, dass man ein Oxiran der Formel II

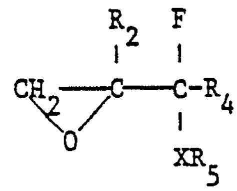

(II)

mit einem Azol der Formel III

M—$R_1$ (III)

zuerst zu einem Alkohol der Formel Ia

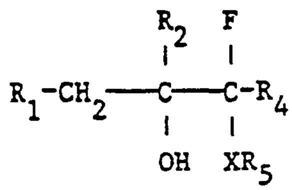

(Ia)

umsetzt und den Alkohol der Formel Ia, sofern ein Ether der Formel I hergestellt werden soll, auf übliche Art, z. B. durch Reaktion mit einer Verbindung der Formel IV

$R_3$—W (IV)

in einen Ether der Formel I überführt, wobei die Substituenten $R_1$ bis $R_5$ in den Formeln Ia, II, III und IV die unter Formel I angegebenen Bedeutungen haben, M für Wasserstoff oder ein Metallatom steht, Hal Halogen bedeutet, und W für OH oder eine übliche Abgangsgruppe steht.

7. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I auf die Pflanze odere deren Standort appliziert.

8. Verwendung von Verbindungen der Formel I gemäss Anspruch 1 zur Bekämpfung und/oder zur präventiven Verhütung eines Befalls von pflanzenschädlichen Mikroorganismen und/oder zur Regulierung des Pflanzenwachstums.

9. Verwendung gemäss Anspruch 8, dadurch gekennzeichnet, dass es sich bei den Mikroorganismen um phytopathogene Pilze handelt.

10. Verwendung gemäss Anspruch 9 gegen Pilze aus den Klassen Ascomycetes, Basidiomycetes oder Fungi imperfecti.

11. Verwendung gemäss Anspruch 8 zur Wuchshemmung im Sinne einer Erhöhung der Knickfestigkeit und Halmverkürzung bei Getreidesorten.

12. Verwendung gemäss Anspruch 11, dadurch gekennzeichnet, dass es sich bei den Getreidesorten um Hafer, Weizen, Gerste oder Roggen handelt.

13. Verwendung gemäss Anspruch 8 zur Wuchshemmung bei Gräsern.

14. Verwendung gemäss Anspruch 8 zur Wuchshemmung bei Bodenbedeckerpflanzen.

15. Verwendung gemäss Anspruch 8 zur Wuchsregulierung bei Leguminosen im Sinne einer Ertragssteigerung.

16. Verwendung gemäss Anspruch 15, dadurch gekennzeichnet, dass es sich um Soja handelt.

17. Verwendung gemäss Anspruch 8 zur Ertragssteigerung durch Seneszenzhemmung bei Getreide, Soja und Baumwolle.

18. Verfahren zur Herstellung der Oxirane der Formel II

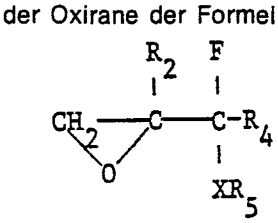

(II)

worin $R_2$, $R_4$ und $R_5$ die in Anspruch 1 unter Formel I angegebenen Bedeutungen haben, dadurch gekennzeichnet, dass man ein Keton der Formel V

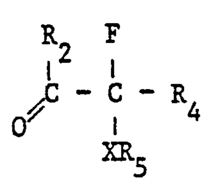

(V)

worin $R_2$, $R_4$ und $R_5$ die unter Formel I gegebene Bedeutung haben, mit Dimethylsulfoniummethylid, Dimethyloxosulfoniummethylid oder einem ihrer Salze in einem polaren, reaktionsinerten Lösungsmittel umsetzt.

**Claims** (for the Contracting States : BE, CH, DE, FR, GB, IT, LI, NL, SE)

1. A compound of the formula

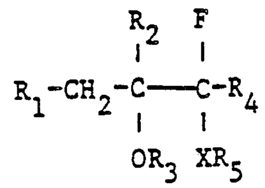

(I)

in which $R_1$ is a 1,2,4-triazolyl or imidazolyl group ; $R_2$ is $C_1$-$C_{12}$-alkyl ; $R_3$ is hydrogen, $C_1$-$C_{12}$-alkyl, $C_2$-$C_4$-alkenyl or $C_1$-$C_4$-alkynyl, or benzyl which is unsubstituted or substituted by $C_1$-$C_6$-alkyl, $C_1$-$C_6$-alkoxy, $C_1$-$C_6$-haloalkyl, halogen and/or cyano ; $R_4$ is hydrogen, fluorine or $C_1$-$C_6$-alkyl ; $R_5$ is an unsubstituted or substituted radical selected from the group consisting of phenyl, naphthyl, biphenyl, benzylphenyl and benzyloxyphenyl, the substituents being selected from the group consisting of halogen, cyano, $C_1$-$C_6$-alkyl, $C_1$-$C_6$-alkoxy, $C_1$-$C_6$-haloalkyl, $C_1$-$C_6$-alkylthio, $C_1$-$C_6$-haloalkoxy, $C_1$-$C_3$-haloalkylthio, nitro and/or thiocyano ; and X is oxygen or sulfur ; or an acid addition salt, a quaternary azolium salt or a metal complex thereof.

2. A compound of the formula I according to claim 1, in which $R_2$ is $C_1$-$C_6$-alkyl ; $R_3$ is hydrogen, $C_1$-$C_6$-alkyl, allyl, propargyl, benzyl, 2,6-dichlorobenzyl, 4-chlorobenzyl, 4-fluorobenzyl, 2,4-dichlorobenzyl or 2-chloro-4-fluorobenzyl ; $R_4$ is hydrogen or methyl ; $R_5$ is phenyl, 4-halophenyl or 2,4-dihalophenyl, or phenyl which is substituted by $CF_3$ ; and X is oxygen or sulfur.

3. A compound of the formula I according to claim 2, in which $R_1$ is 1,2,4-triazole ; $R_2$ is tert-butyl or isopropyl ; $R_3$ is hydrogen or $C_1$-$C_5$-alkyl ; $R_4$ is hydrogen or methyl ; $R_5$ is 4-halophenyl, 2,4-dihalophenyl or $4-CF_3-C_6H_4-$ ; and X is oxygen.

4. A compound of the formula I according to claim 3, in which $R_3$ is hydrogen and the other substituents are as defined in claim 3.

5. A compound of the formula I according to claim 1, selected from the group : 1-fluoro-1-(4-chlorophenoxy)-2-(1H-1,2,4-triazol-1'-ylmethyl)-3,3-dimethylbutan-2-ol, 1-fluoro-1-(4-fluorophenoxy)-2-(1H-1,2,4-triazol-1'-ylmethyl)-3,3-dimethylbutan-2-ol, 1-fluoro-1-(4-chlorophenoxy)-2-(1H-imidazol-1'-ylmethyl)-3,3-dimethylbutan-2-ol and 1-fluoro-1-(2,4-dichlorophenoxy)-2-(1H-1,2,4-triazol-1'-ylmethyl)-3,3-dimethylbutan-2-ol.

6. A process for the preparation of a compound of the formula I as defined in claim 1, which comprises first reacting an oxirane of the formula II

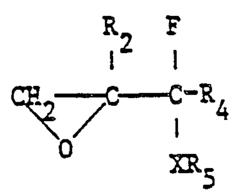

(II)

with an azole of the formula III

$$M—R_1 \qquad (III)$$

to give an alcohol of the formula Ia

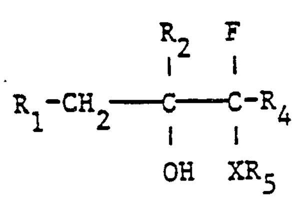

(Ia)

and, if an ether of the formula I is to be prepared, converting the alcohol of the formula Ia into an ether of the formula I in conventional manner, for example by reaction with a compound of the formula IV

$$R_3\text{—}W \qquad (IV)$$

in which formulae Ia, II, III and IV the substituents $R_1$ to $R_5$ are as defined for formula I, M is hydrogen or a metal atom, Hal is halogen and W is OH or a conventional leaving group.

7. A composition for controlling microorganisms or preventing attack by said microorganisms and/or for regulating plant growth, which composition contains, as at least one active component, a compound of the formula I according to claim 1.

8. A method of controlling phytopathogenic organisms or protecting cultivated plants from attack by said phytopathogenic microorganisms and/or of regulating plant growth, which method comprises applying a compound of the formula I as defined according to claim 1 to the plants or to the locus thereof.

9. Use of a compound of the formula I according to claim 1 for controlling phytopathogenic microorganisms and/or preventing attack by said phytopathogenic microorganisms and/or for regulating plant growth.

10. Use according to claim 9, wherein the microorganisms are phytopathogenic fungi.

11. Use according to claim 10 against fungi of the classes Ascomycetes, Basidiomycetes or Fungi imperfecti.

12. Use according to claim 9 for growth inhibition for the purpose of increasing resistance to lodging and of shortening the stem in cereal crops.

13. Use according to claim 12, wherein the cereal crops are oats, wheat, barley or rye.

14. Use according to claim 9 for inhibiting the growth of grasses.

15. Use according to claim 9 for inhibiting the growth of cover crops.

16. Use according to claim 9 for regulating the growth of leguminosae in order to increase the yield.

17. Use according to claim 16 on soybeans.

18. Use according to claim 16 for increasing the yield by inhibition of senescence in cereals, soybeans and cotton.

19. An oxirane of the formula II

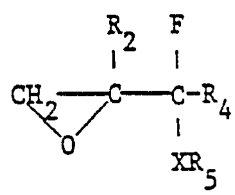

(II)

in which $R_2$, $R_4$ and $R_5$ are as defined for formula I in claim 1.

**Claims** (for the Contracting State AT)

1. A composition for controlling microorganisms or preventing attack by said microorganisms and/or for regulating plant growth, which composition contains, as active component, at least one compound of the formula I

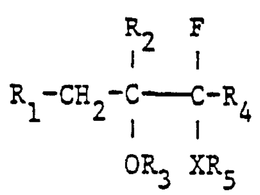

(I)

in which $R_1$ is a 1,2,4-triazolyl or imidazolyl group ; $R_2$ is $C_1$-$C_{12}$-alkyl ; $R_3$ is hydrogen, $C_1$-$C_{12}$-alkyl, $C_2$-$C_4$-alkenyl or $C_1$-$C_4$-alkynyl, or benzyl which is unsubstituted or substituted by $C_1$-$C_6$-alkyl, $C_1$-$C_6$-alkoxy, $C_1$-$C_6$-haloalkyl, halogen and/or cyano ; $R_4$ is hydrogen, fluorine or $C_1$-$C_6$-alkyl ; $R_5$ is an unsubstituted or substituted radical selected from the group consisting of phenyl, naphthyl, biphenyl, benzylphenyl and benzyloxyphenyl, the substituents being selected from the group consisting of halogen, cyano, $C_1$-$C_6$-alkyl, $C_1$-$C_6$-alkoxy, $C_1$-$C_6$-haloalkyl, $C_1$-$C_6$-alkylthio, $C_1$-$C_6$-haloalkoxy, $C_1$-$C_3$-haloalkylthio, nitro and/or thiocyano ; and X is oxygen or sulfur ; or an acid addition salt, a quaternary azolium salt or a metal complex thereof.

2. A composition according to claim 1, in which $R_2$ is $C_1$-$C_6$-alkyl ; $R_3$ is hydrogen, $C_1$-$C_6$-alkyl, allyl, propargyl, benzyl, 2,6-dichlorobenzyl, 4-chlorobenzyl, 4-fluorobenzyl, 2,4-dichlorobenzyl or 2-chloro-4-fluorobenzyl ; $R_4$ is hydrogen or methyl ; $R_5$ is phenyl, 4-halophenyl or 2,4-dihalophenyl, or phenyl which is substituted by $CF_3$ ; and X is oxygen or sulfur.

3. A composition according to claim 2, in which $R_1$ is 1,2,4-triazole ; $R_2$ is tert-butyl or isopropyl ; $R_3$ is hydrogen or $C_1$-$C_5$-alkyl ; $R_4$ is hydrogen or methyl ; $R_5$ is 4-halophenyl, 2,4-dihalophenyl or

$4-CF_3-C_6H_4-$ ; and X is oxygen.

4. A composition according to claim 3, in which $R_3$ is hydrogen and the other substituents are as defined in claim 3.

5. A composition according to claim 1, which contains a compound selected from the group : 1-fluoro-1-(4-chlorophenoxy)-2-(1H-1,2,4-triazol-1'-ylmethyl)-3,3-dimethylbutan-2-ol, 1-fluoro-1-(4-fluorophenoxy-2-(1H-1,2,4-triazol-1'-ylmethyl)-3,3-dimethylbutan-2-ol, 1-fluoro-1-(4-chlorophenoxy)-2-(1H-imidazol-1'-ylmethyl)-3,3-dimethylbutan-2-ol and 1-fluoro-1-(2,4-dichlorophenoxy)-2-(1H-1,2,4-triazol-1'-ylmethyl)-3,3-dimethylbutan-2-ol.

6. A process for the preparation of a compound of the formula I as defined in claim 1, which comprises first reacting an oxirane of the formula II

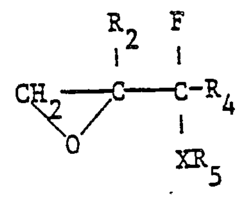

(II)

with an azole of the formula III

$$M-R_1 \qquad (III)$$

to give an alcohol of the formula Ia

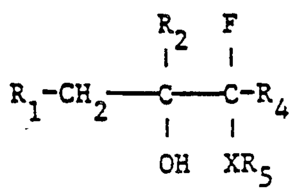

(Ia)

and, if an ether of the formula I is to be prepared, converting the alcohol of the formula Ia into an ether of the formula I in conventional manner, for example by reaction with a compound of the formula IV

$$R_3-W \qquad (IV)$$

in which formulae Ia, II, III and IV, the substituents $R_1$ to $R_5$ are as defined for formula I, M is hydrogen or a metal atom, Hal is halogen and W is OH or a conventional leaving group.

7. A method of controlling phytopathogenic organisms or protecting cultivated plants from attack by said phytopathogenic microorganisms and/or of regulating plant growth, which method comprises applying a compound of the formula I as defined according to claim 1 to the plants or to the locus thereof.

8. Use of a compound of the formula I according to claim 1 for controlling phytopathogenic microorganisms and/or preventing attack by said phytopathogenic microorganisms and/or for regulating plant growth.

9. Use according to claim 8, wherein the microorganisms are phytopathogenic fungi.

10. Use according to claim 9 against fungi of the classes Ascomycetes, Basidiomycetes or Fungi imperfecti.

11. Use according to claim 8 for growth inhibition for the purpose of increasing resistance to lodging and of shortening the stem in cereal crops.

12. Use according to claim 11, wherein the cereal crops are oats, wheat, barley or rye.

13. Use according to claim 8 for inhibiting the growth of grasses.

14. Use according to claim 8 for inhibiting the growth of cover crops.

15. Use according to claim 8 for regulating the growth of leguminosae in order to increase the yield.

16. Use according to claim 15 on soybeans.

17. Use according to claim 8 for increasing the yield by inhibition of senescence in cereals, soybeans and cotton.

18. A process for the preparation of an oxirane of the formula II

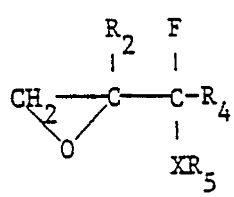

(II)

in which $R_2$, $R_4$ and $R_5$ are as defined for formula I in claim 1, which process comprises reacting a ketone of the formula V

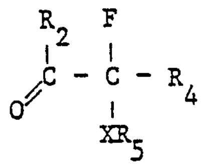

(V)

in which $R_2$, $R_4$ and $R_5$ are as defined for formula I, with dimethylsulfonium methylide, dimethyloxosulfonium methylide or with a salt thereof, in a polar inert solvent.

**Revendications** (pour les Etats contractants : BE, CH, DE, FR, GB, IT, LI, NL, SE)

1. Composés de formule I

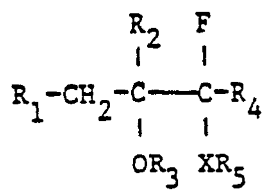

(I)

où $R_1$ représente un 1,2,4-triazolyl- ou imidazolyle ; $R_2$ représente un alcoyle en $C_1$-$C_{12}$ ; $R_3$ représente un hydrogène, un alcoyle en $C_1$-$C_{12}$, alcényle en $C_2$-$C_4$, alcinyle en $C_1$-$C_4$ ou benzyle éventuellement substitué par un alcoyle en $C_1$-$C_6$, alcoxy en $C_1$-$C_6$, haloalcoyle en $C_1$-$C_6$, halogène et/ou cyano ; $R_4$ représente un hydrogène, fluor ou alcoyle en $C_1$-$C_6$ ; $R_5$ représente un radical non substitué ou substitué, choisi dans la série phényle, naphtyle, biphényle, benzylphényle et benzyloxyphényle, où les substituants sont choisis dans la série halogène, cyano, alcoyle en $C_1$-$C_6$, alkoxy en $C_1$-$C_6$, haloalcoyle en $C_1$-$C_6$, alcoylthio en $C_1$-$C_6$, haloalkoxy en $C_1$-$C_6$, haloalcoylthio en $C_1$-$C_3$, nitro et/ou rhodano ; et X représente un oxygène ou un soufre ; y compris les sels d'addition d'acide, les sels d'azolium quaternaires et complexes métalliques.

2. Composés de formule I selon la revendication 1, où $R_2$ représente un alcoyle en $C_1$-$C_6$ ; $R_3$ représente un hydrogène, alcoyle en $C_1$-$C_6$, l'allyle, propargyle, benzyle, 2,6-dichlorobenzyle, 4-chlorobenzyle, 4-fluorobenzyle, 2,4-dichlorobenzyle ou 2-chloro-4-fluorobenzyle ; $R_4$ représente un hydrogène de méthyle, $R_5$ représente un phényle, 4-halophényl, 2,4-dialophényl ou un phényle substitué par $CF_3$ et X représente un oxygène ou un soufre.

3. Composés de formule I selon la revendication 2 où $R_1$ représente 1,2,4-triazole ; $R_2$ représente un tert.-butyl ou isopropyl, $R_3$ représente un hydrogène ou un alcoyle en $C_1$-$C_4$ ; $R_4$ représente un hydrogène ou un méthyle, $R_5$ représente un 4-halophényle, 2,4-dihalophényle ou $4—CF_3—C_6H_4—$ et X représente un oxygène.

4. Composés de formule I selon la revendication 3 où $R_3$ représente un hydrogène et les autres substituants sont définis comme dans la revendication 3.

5. Composés de formule I selon la revendication 1, choisis dans la série : 1-fluoro-1-(4-chlorophénoxy)-2-(1H-1,2,4-triazol-1'-ylméthyl)-3,3-diméthyl-butan-2-ol, 1-fluoro-1-(4-fluorophénoxy)-2-(1H-1,2,4-triazol-1'-ylméthyl)-3,3-diméthyl-butan-2-ol, 1-fluoro-1-(4-chlorophénoxy)-2-(1H-imidazol-1'-ylméthyl)-3,3-diméthyl-butan-2-ol, 1-fluoro-1-(2,4-dichlorophénoxy)-2-(1H-1,2,4-triazol-1'-ylméthyl)-3,3-diméthylbutan-2-ol.

6. Procédé de préparation des composés de formule I définis dans la revendication 1, caractérisé en ce qu'on fait tout d'abord réagir un oxiranne de formule II

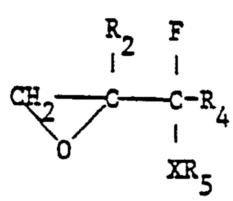

(II)

avec un azol de formule III

$M—R_1$ (III)

pour donner un alcool de formule Ia

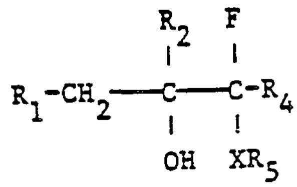

(Ia)

et en ce qu'on transforme l'alcool de formule Ia, dans la mesure où l'on doit préparer un éther de formule I, de manière habituelle, et par réaction avec un composé de formule IV

$$R_3—W \quad (IV)$$

en un éther de formule I, où les substituants $R_1$ à $R_5$ dans les formules Ia, II, III et IV ont les significations données sous la formule I, M représente un hydrogène ou un atome métallique, Hal représente un halogène, et W représente OH ou un groupe sortant habituel.

7. Agent de lutte ou de prévention d'une attaque par les micro-organismes et/ou de régulation de la croissance végétale, caractérisé en ce qu'il contient au moins un composant actif, composé de formule I selon la revendication 1.

8. Procédé de lutte ou de prévention d'une attaque des plantes cultivées par les micro-organismes phytopathogènes ou de régulation de la croissance végétale, caractérisé en ce qu'on applique un composé de formule I défini selon la revendication 1 sur les plantes ou l'endroit où il se trouve.

9. Application de composés de formule I selon la revendication 1 à la lutte et/ou à la prévention d'une attaque par des micro-organismes causant des dommages aux plantes et/ou à la régulation de la croissance végétale.

10. Application selon la revendication 9, caractérisée en ce qu'il s'agit pour les micro-organismes de champignons phytopathogènes.

11. Application selon la revendication 10 contre les champignons des classes Ascomycetes, Basidiomycetes ou Fungi imperfecti.

12. Application selon la revendication 9, à l'inhibition de la croissance au sens d'un accroissement de la résistance au tallage et d'un raccourcissement des tiges dans les variétés de céréales.

13. Application selon la revendication 12, caractérisée en ce qu'il s'agit quant aux variétés de céréales de l'avoine, le blé, l'orge ou le seigle.

14. Application selon la revendication 9 à l'inhibition de la croissance des graminées.

15. Application selon la revendication 9, à l'inhibition de la croissance des plantes protectrices du sol.

16. Application selon la revendication 9, à la régulation de la croissance des légumineuses au sens d'un accroissement du rendement.

17. Application selon la revendication 16, caractérisée en ce qu'il s'agit du soja.

18. Application selon la revendication 16, pour l'accroissement du rendement par l'inhibition de la sénescence dans les céréales, le soja et le coton.

19. Oxirane de formule II

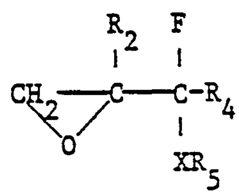

(II)

où $R_2$, $R_4$ et $R_5$ ont les significations données dans la revendication 1 sous la formule I.

**Revendications** (pour l'Etat contractant AT)

1. Agent de lutte ou de prévention d'une attaque par des micro-organismes et/ou régulation de la croissance végétale, caractérisé en ce qu'il contient comme composant actif au moins un composé de Formule I

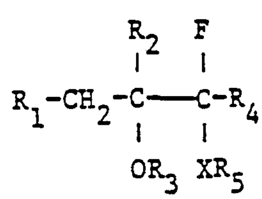

(I)

où $R_1$ représente un 1,2,4-triazolyl- ou imidazolyle ; $R_2$ représente un alcoyle en $C_1$-$C_{12}$ ; $R_3$ représente un hydrogène, un alcoyle en $C_1$-$C_{12}$, alcényle en $C_2$-$C_4$, alcinyle en $C_2$-$C_4$ ou benzyle éventuellement substitué par un alcoyle en $C_1$-$C_6$, alcoxy en $C_1$-$C_6$, haloalcoyle en $C_1$-$C_6$, halogène et/ou cyano ; $R_4$ représente un hydrogène, fluor ou alcoyle en $C_1$-$C_6$ ; $R_5$ représente un radical non substitué ou substitué, choisi dans la série phényle, naphtyle, biphényle, benzylphényle et benzyloxyphényle, où les substituants sont choisis dans la série halogène, cyano, alcoyle en $C_1$-$C_6$, alkoxy en $C_1$-$C_6$, haloalcoyle en $C_1$-$C_6$, alcoylthio en $C_1$-$C_6$, haloalkoxy en $C_1$-$C_6$, haloalcoylthio en $C_1$-$C_3$, nitro et/ou rhodano ; et X représente un oxygène ou un soufre ; y compris les sels d'addition d'acide, les sels d'azolium quaternaires et complexes métalliques.

2. Agent selon la revendication 1, où $R_2$ représente un alcoyle en $C_1$-$C_6$ ; $R_3$ représente un hydrogène, alcoyle en $C_1$-$C_6$, l'allyle, propargyle, benzyle, 2,6-dichlorobenzyle, 4-chlorobenzyle, 4-fluorobenzyle, 2,4-dichlorobenzyle ou 2-chloro-4-fluorobenzyle ; $R_4$ représente un hydrogène de méthyle, $R_5$ représente un phényle, 4-halophényle, 2,4-dialophényl ou un phényle substitué par $CF_3$ et X représente un oxygène ou un soufre.

3. Agent selon la revendication 2, où $R_1$ représente 1,2,4-triazole ; $R_2$ représente un tert.-butyle ou un isopropyle, $R_3$ représente un hydrogène ou un alcoyle en $C_1$-$C_5$ ; $R_4$ représente un hydrogène ou un méthyle ; $R_5$ représente un 4-halophényle, 2,4-dihalophényle ou $4—CF_3—C_6H_4—$ et X représente un oxygène.

4. Agent selon la revendication 3 où $R_3$ représente un hydrogène et les autres substituants sont définis comme dans la revendication 3.

5. Agent selon la revendication 1, caractérisé en ce qu'il contient une matière active de la série 1-fluoro-1-(4-chlorophénoxy)-2-(1H-1,2,4-triazol-1'-ylméthyl)-3,3-diméthyl-butan-2-ol, 1-fluoro-1-(4-fluorophénoxy)-2-(1H-1,2,4-triazol-1'-ylméthyl)-3,3-diméthyl-butan-2-ol, 1-fluoro-1-(4-chlorophénoxy)-2-(1H-imidazol-1'-ylméthyl)-3,3-diméthyl-butan-2-ol, 1-fluoro-1-(2,4-dichlorophénoxy)-2-(1H-1,2,4-triazol-1'-ylméthyl)-3,3-diméthylbutan-2-ol.

6. Procédé de préparation des composés de formule I définis dans la revendication 1, caractérisé en ce qu'on fait tout d'abord réagir un oxirane de formule II

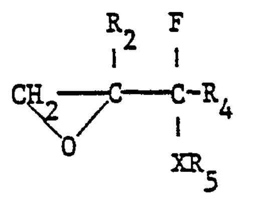

(II)

avec un azol de formule III

$$M—R_1 \qquad (III)$$

pour donner un alcool de formule Ia

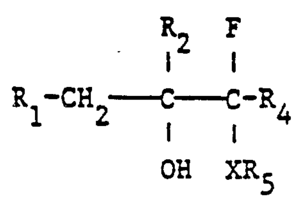

(Ia)

et en ce qu'on transforme l'alcool de formule Ia, dans la mesure où l'on doit préparer un éther de formule I, de manière habituelle, et par réaction avec un composé de formule IV

$$R_3—W \qquad (IV)$$

en un éther de formule I, où les substituants $R_1$ à $R_5$ dans les formules Ia, II, III et IV ont les significations données sous la formule I, M représente un hydrogène ou un atome métallique, Hal représente un halogène, et W représente OH ou un groupe sortant habituel.

7. Procédé de lutte ou de prévention d'une attaque contre les plantes cultivées par les micro-organismes et/ou de régulation de la croissance végétale, caractérisé en ce qu'on applique un composé de formule I selon la revendication 1 sur les plantes ou l'endroit où elles se trouvent.

8. Application de composés de formule I selon la revendication 1 à la lutte et/ou à la prévention d'une attaque par des micro-organismes causant des dommages aux plantes et/ou à la régulation de la croissance végétale.

9. Application selon la revendication 8, caractérisée en ce qu'il s'agit pour les micro-organismes de champignons phytopathogènes.

10. Application selon la revendication 10 contre les champignons des classes Ascomycetes, Basidiomycetes ou Fungi imperfecti.

11. Application selon la revendication 8, à l'inhibition de la croissance au sens d'un accroissement de la résistance au tallage et d'un raccourcissement des tiges dans les variétés de céréales.

12. Application. selon la revendication 11, caractérisée en ce qu'il s'agit quant aux variétés de céréales de l'avoine, le blé, l'orge ou le seigle.

13. Application selon la revendication 8 à l'inhibition de la croissance des graminées.

14. Application selon la revendication 8, à l'inhibition de la croissance des plantes protectrices du sol.

15. Application selon la revendication 8, à la régulation de la croissance des légumineuses au sens d'un accroissement du rendement.

16. Application selon la revendication 15, caractérisée en ce qu'il s'agit du soja.

17. Application selon la revendication 8, pour l'accroissement du rendement par l'inhibition de la sénescence dans les céréales, le soja et le coton.

18. Procédé de préparation des oxiranes de formule II

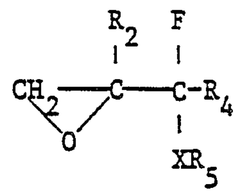

(II)

où $R_2$, $R_4$ et $R_5$ ont les significations données dans la revendication 1 sous la formule I, caractérisé en ce qu'on fait réagir une cétone de formule V

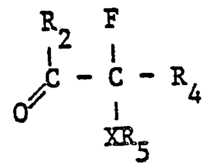

(V)

où $R_2$, $R_4$, $R_5$ ont la signification donnée sous la formule I, avec du méthylure de diméthylsulfonium, du méthylure de diméthyloxosulfonium ou un de leurs sels dans un solvant polaire inerte vis-à-vis de la réaction.